# EUROPEAN PATENT APPLICATION

(11) **EP 1 133 961 A1**
(43) Date of publication of application: **19.09.2001**
(21) Application number: 00200935.5
(22) Date of filing: 15.03.2000
(51) Int. Cl.: A61F 13/15

(54) **Sanitary napkin or panty liner for use with a tanga undergarment**

(71) Applicant: The Procter & Gamble Company, Cincinnati, Ohio 45202 (US)
(72) Inventor: Schäfer, Jochen, 65824 Schwalbach (DE); Will, Susanne, 65830 Kriftel (DE); Kitzinger, Ulrike Dagmar Frauke, 65529 Waldems (DE); Renz, Anke Simone, 61449 Steinbach (DE)
(74) Representative: Hirsch, Uwe Thomas

(57) **Abstract**

The present invention relates to a breathable absorbent thong shaped sanitary napkin or panty liner. Such sanitary napkins or panty liners are particularly useful for wearing in so-called thong slips or G-string undergarments. According to the present invention the sanitary napkins or panty liners are provided with a water vapour permeable, preferably an air permeable, backsheet for breathability. The absorbent core of such sanitary napkins or panty liners comprises in addition to a fibrous matrix at lest one fibrous active material selected from fibrous superabsorbent material or fibrous odour control material or combinations thereof.

## Description

### Field of the invention

The present invention relates to a breathable absorbent thong shaped sanitary napkin or panty liner. Such sanitary napkins or panty liners are particularly useful for wearing in so-called thong slips or G-string undergarments. According to the present invention the sanitary napkins or panty liners are provided with a water vapour permeable, preferably an air permeable apertured, backsheet for breathability. The absorbent core of such sanitary napkins or panty liners comprises in addition to a fibrous matrix a fibrous super-absorbent material or fibrous odour control material.

### Background of the invention

The use of water vapour permeable, preferably air permeable apertured, backsheets for the breathability of disposable absorbent articles is well-known in the art. For example European patent applications EP-710 471 or EP- 710 472 disclose apertured film backsheets used in conjunction with fibrous non-woven materials for breathability and improved soil-through performance.

Both disclosures also mention the benefit of super-absorbent material however without disclosing or considering fibrous super-absorbent material.

On the other hand fibrous super-absorbent material has been mentioned for example in EP-A-0 336 578 which relates specifically to thin flexible sanitary napkins comprising optionally fibrous super-absorbent materials. This disclosure however lacks consideration of breathability and more specific it does not disclose apertures in the backsheets.

W0-A-92/07534 relates and discloses fibrous super-absorbent structures. However it fails to consider breathability of disposable absorbent articles as such and in particular does not mention apertured backsheets in conjunction with the use of the absorbent structures in disposable absorbent article.

European patent application EP-682 927, entitled "Shields against absorbent particle loss in absorbent products" does address the problem of particle migration trough apertures in a polymer film. However this disclosure relates specifically to an aperture topsheet but not a breathable aperture backsheet. Also this disclosure solves the problem of particle migration through an apertured polymer film by including a barrier between the particle and the aperture, i.e. the use of a shield.

An attempt to address the problem of particle loss by sealing off an edge area of a laminate with particles comprised between 2 fibrous layers has been made in WO 98/24620, WO 98/28128 and EP-875 225. The disclosed embodiments do, however, create a region along the cut line in which the layers of the laminate are joined to each other so as to prevent the particle loss. The seal as such does therefore create a region of different product characteristics then the remainder of the structure such as for example stiffness, harshness or, if adhesive is used for the seal, stickiness.

Of course the same kind of problems arises if another particular material is used in the context of absorbent articles having an apertured topsheet or an apertured backsheet or if the particular material is contained in a laminate. Especially odour control materials such as zeolites, silicate, carbon black, silica-gel particles are well known as odour control ingredients for sanitary napkins or panty liners. But independent for which purpose a particle material is used as a component of the absorbent structure of an absorbent article they can all benefit from the same isolation systems as disclosed in the above patents for super absorbent particles.

It now has been found that in the context of a newly designed sanitary napkin or panty liner, namely one with a thong shape, the isolation systems that historically have been applied to sanitary napkins or panty liners in order to prevent the loss or migration of particles to the outside of the article cannot be used anymore. The reason is the peculiar shape of these thong shaped sanitary napkins or panty liners which are designed to fit into the crotch region, (this is the region with a certain extend towards the front and rear of the center of an undergarment), of undergarments which are known as thong slips, G-string undergarments, tanga slips, string panties or simply strings. The thong shape for such thong slips is substantially triangular with a wider front and a narrower rear portion. Such articles are worn in very close proximity to the body of a user especially in the region of their narrow width side, i.e. towards the rear. Particularly well fitting embodiments of this thong shaped sanitary napkin or panty liner will eventually even fold partially into the creases between the buttocks during use.

It is therefore necessary to provide such thong shaped sanitary napkins or panty liners without any irritation causing material or design feature especially in a peripheral rim region. It is further highly desirable to provide the thong shaped sanitary napkins or panty liners with an absorbent capacity which is similar to the overall absorbent capacity provided by the substantially larger traditional sanitary napkins or panty liners. In order to provide this the absorbency per area needs to be particularly high for such thong shaped sanitary napkins or panty liners. Finally, the close proximity of such thong shaped sanitary napkins or panty liners is causing an occlusion to air permeation which conventionally occurs by frequent separation of the article from the body forming gaps between the article and the skin of the wearer. It is from this aspect particularly important to provide such thong shaped sanitary napkins or panty liners with breathability which allows perspiration liquid or absorbed liquid to dissipate by vaporization, preferably even by providing an air exchange through the article itself (rather than between the article and the skin of the wearer).

Based on the above the present invention addresses the problem of preventing a possible particle loss of active material, such as super-absorbent or odour control material, through a breathable backsheet or the periphery of an absorbent core in the context of the comfort requirements of thong shaped sanitary napkins or panty liners. It is apparent that an article solving this problem will also need to satisfy the conditions of usefulness in the context of manufacturing a commodity mass product such as high-speed making (at a linear production speed of at least 9 km/hour), low cost material availability, environmental concerns such as material composition and material/energy consumption.

### Brief description of the drawings

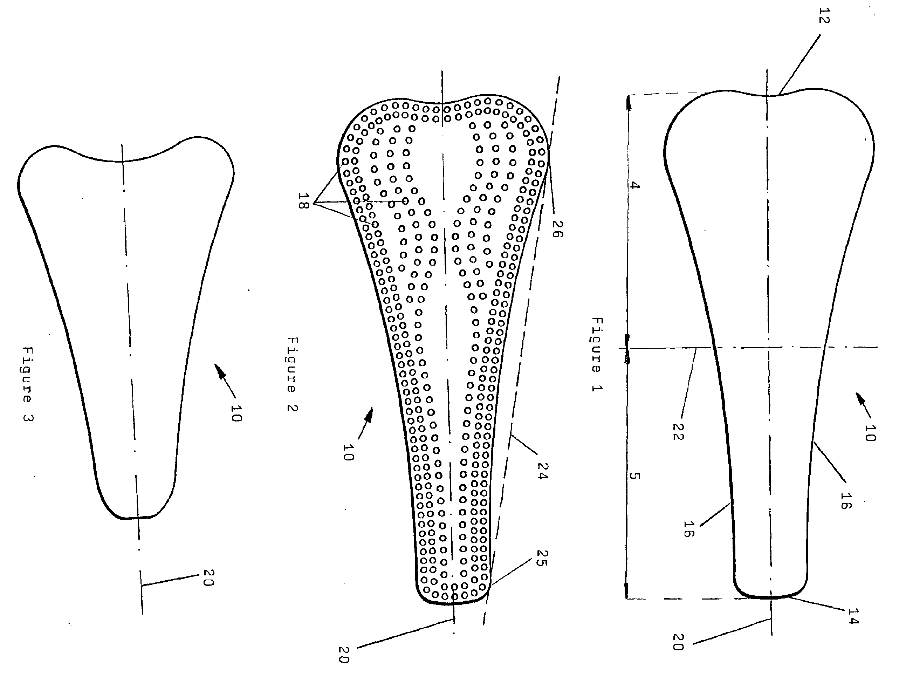

Figures 1, 2 and 3 show 3 alternative top plan views of thong shaped sanitary napkins or panty liners.

### Brief description of the invention

The present invention relates to thong shaped disposable, breathable, absorbent sanitary napkins or panty liners which have an absorbent core which comprises a fibrous matrix and further at least 5% by weight of a fibrous super absorbent material, and optionally a fibrous odour control material. The thong shaped absorbent sanitary napkin or panty liner has a front end and a rear end and longitudinal side edges. Front end, rear end and side edges together forming the periphery of the sanitary napkin or panty liner. The thong shaped sanitary napkin or panty liner further has a wearer facing surface directed towards the wearer during use and a garment facing surface directed towards an undergarment (unusually a thong slip) during use. The sanitary napkin or panty liner has a thong shape which is such that the front of the sanitary napkin or panty liner is at least twice as wide as the rear and the longitudinal side edges connecting the front and rear end of the sanitary napkin or panty liner have a linear or inwardly concave contour. Besides the absorbent core the sanitary napkin or panty liner also comprises a backsheet on the garment facing surface to prevent absorbed liquid from passing through and soil the undergarment. The backsheet is breathable by being water vapour permeable, preferably air permeable.

Preferably, the sanitary napkin or panty liner also comprises on its garment facing surface a panty fastening adhesive which is covering at least 75 % of the surface of the backsheet, preferably 85 % of the surface of the backsheet. The panty fastening adhesive needs to be provided in a dispersed fashion (random or designed) or it needs to be water vapour pervious in order to support the breathability of the backsheet. Most preferably the panty adhesive covers the complete portion of the sanitary napkin or panty liner which forms the rearward half of the sanitary napkin or panty liner (splitting the napkin or panty liner in transverse direction at half of its longitudinal length).

In another preferred embodiment according to the present invention possibly in connection with the previously preferred embodiment of the present invention the absorbent core of the sanitary napkin or panty liner extends to the periphery of both longitudinal side edges of the sanitary napkin or panty liner and the rear end. Even more preferably the absorbent core extends completely throughout the sanitary napkin or panty liner such that the periphery of the core and the periphery of the sanitary napkin or panty liner are identical.

It is further generally preferred that the sanitary napkin or panty liner also comprises a liquid and water vapour pervious topsheet especially at least in its central portion an apertured thermo-plastic film topsheet. Finally, the sanitary napkin or panty liner is preferably very thin for comfort, pliability and flexibility such that its thickness is less than 2.54 mm or preferably even less than 2.032 mm.

### Detailed description of the invention

### Definitions

As used herein, the term "body fluids" includes perspiration, urine, menses and vaginal discharges.

As used herein, the terms "sanitary napkin" and "panty liner" refer to an absorbent article that is worn by females adjacent to the pudendal region, generally external to the urogenital region, and which is intended to absorb and contain aqueous body fluids and other vaginal discharges from the wearer's body.

As used herein, the term "thong liner" refers to sanitary napkins or panty liners which are capable of absorbing aqueous body fluids and are designed to fit into thong slips or string undergarments as defined above.

As used herein the term "granular" refers to materials with particles of uniform size or having a size distribution in which at lest 5 % by weight of the particles have a largest diameter of at least 0.2 mm. The term "non-granular" excludes those materials which are granular.

As used herein the term "transverse " refers to a direction which connects the right and the left leg opening of an undergarment. In relation to thong liners transverse refers to a direction of a component of a thong liner which is generally parallel to the transverse direction during intended use of the thong liner. As used herein the term "longitudinal" generally refers to a direction perpendicular to the transverse direction.

As used herein the term "wearer-facing" surface refers to the surface of a component of a thong liner generally oriented to face the wearer during use of the thong liner. As used herein the term "garment facing" surface refers to the opposite surface than the wearer facing surface.

Unless otherwise noted in the following all percentiles refer to percent by weight.

### Detailed description of the elements of thong liners according to the invention

According to the present invention the thong liner comprises two main elements: a core and a backsheet. Typically the thong liner also comprises a topsheet which is worn next to the skin of the user while the backsheet is worn next to the undergarment of the user with the core interposed between both. Each of the elements of the thong liner can be selected from a wide variety of alternatives an comprise several elements and/or layers contributing to the individual function of each elements.

In the following the elements of the thong liner are described.

### Absorbent core

The absorbent core can be a single entity or comprise several layers. It can includes the following components: (a) optionally a primary fluid distribution layer; (b) optionally a secondary fluid distribution layer; (c) a fluid storage layer; (d) optionally a fibrous layer underlying the storage layer; and (e) other optional components.

### a. Primary Fluid Distribution Layer

One optional component of the absorbent core according to the present invention is the primary fluid distribution layer. This primary distribution layer typically underlies the topsheet (if present) and is in fluid communication therewith. The primary distribution layer acquires body fluid for ultimate distribution to the storage layer. This transfer of fluid through the primary distribution layer occurs mainly in the thickness, but may also provide distribution along the longitudinal and transverse directions of the thong liner.

### b. Optional Secondary Fluid Distribution Layer

Also optional according to the present invention is a secondary fluid distribution layer. This secondary distribution layer typically underlies the primary distribution layer and is in fluid communication therewith. The purpose of this secondary distribution layer is to readily acquire bodily fluid from the primary distribution layer and distribute it along the longitudinal and transverse directions of the thong liner before transfer to the underlying storage layer. This helps the fluid capacity of the underlying storage layer to be fully utilized.

### c. Fluid Storage Layer

Positioned in fluid communication with , and typically underlying the primary or secondary distribution layers, is a fluid storage layer. It comprises non-granular super-absorbent gelling materials usually referred to as "hydrogels," "superabsorbent" "hydrocolloid" materials. Absorbent gelling materials are those materials that, upon contact with aqueous fluids, especially body fluids, imbibes such fluids and thus form hydrogels. These absorbent gelling materials are typically capable of absorbing large quantities of aqueous body fluids, and are further capable of retaining such absorbed fluids under moderate pressures. In the prior art these absorbent gelling materials are typically in a granular form of discrete, nonfibrous particles. However, according to the present invention these super-absorbent gelling materials are provided in non-granular form, preferably in a fibrous form.

In the fluid storage layer these absorbent gelling materials can be dispersed homogeneously or non-homogeneously in a suitable fibrous matrix also referred to as carrier. Suitable carriers include cellulose fibers, in the form of fluff, such as is conventionally utilized in absorbent cores. Modified cellulose fibers such as the stiffened cellulose fibers can also be used. Synthetic fibers can also be used and include those made of cellulose acetate, polyvinyl fluoride, polyvinylidene chloride, acrylics (such as Orlon), polyvinyl acetate, non-soluble polyvinyl alcohol, polyethylene, polypropylene, polyamides (such as nylon), polyesters, bicomponent fibers, tricomponent fibers, mixtures thereof and the like. Preferred synthetic and man-made fibers have a denier of from about 3 denier per filament to about 25 denier per filament, more preferably from about 5 denier per filament to about 16 denier per filament. Also preferably, the fiber surfaces are hydrophilic or are treated to be hydrophilic. The storage layer can also include filler materials, such as Perlite, diatomaceous earth, Vermiculite, etc., that lower rewet problems, however, only in a non-granular form. Further the storage layer may comprise a binder including but not limited to Latex binders which can be sprayed as an aqueous solution onto the surface of the storage layer prior to curing.

If the absorbent gelling materials are dispersed non-homogeneously in a fibrous matrix, the storage layer can be locally homogeneous, i.e. have a distribution gradient in one or several directions within the dimensions of the storage layer. Non-homogeneous distribution thus includes e.g. laminates of the fibrous carriers enclosing the non-granular absorbent gelling materials.

Generally, the storage layer comprises from 5 % to 95 % absorbent gelling materials, preferably from 5 % to 50 %, most preferably from 8 % to 35 %, absorbent gelling materials. Further the storage layer can comprise from 5 % to 95 % carrier fibers, preferably from 95 % to 50 %, most preferably from 92 % to 65 % carrier fibers.

Suitable absorbent gelling materials for use herein will most often comprise a substantially water-insoluble, slightly crosslinked, partially neutralized, polymeric gelling material. This material forms a hydrogel upon contact with water. Such polymer materials can be prepared from polymerizable, unsaturated, acid-containing monomers. Suitable unsaturated acidic monomers for use in preparing the polymeric absorbent gelling material used in this invention include those listed in U.S. Patent 4,654,039 (Brandt et al), issued March 31, 1987, and reissued as RE 32,649 on April 19, 1988. Preferred monomers include acrylic acid, methacrylic acid, and 2-acrylamido-2-methyl propane sulfonic acid. Acrylic acid itself is especially preferred for preparation of the super-absorbent material. The polymeric component formed from the unsaturated, acid-containing monomers can be grafted onto other types of polymer moieties such as starch or cellulose. Polyacrylate grafted starch materials of this type are especially preferred. Preferred polymeric absorbent gelling materials that can be prepared from conventional types of monomers include hydrolyzed acrylonitrile grafted starch, polyacrylate grafted starch, polyacrylates, maleic anhydride-based copolymers and combinations thereof. Especially preferred are the polyacrylates and polyacrylate grafted starch.

Whatever the nature of the basic polymer components of the hydrogel-forming polymeric absorbent gelling materials, such materials will in general be slightly crosslinked. Crosslinking serves to render the hydrogel-forming polymer gelling materials substantially water-insoluble, and cross-linking thus in part determines the gel volume and extractable polymer characteristics of the hydrogels formed from these polymeric gelling materials. Suitable crosslinking agents are well known in the art and include, for example, those described in greater detail in U.S. Patent 4,076,663 (Masuda et al), issued February 28, 1978. Preferred crosslinking agents are the di- or polyesters of unsaturated mono- or polycarboxylic acids with polyols, the bisacrylamides and the di- or triallyl amines. Other preferred crosslinking agents are N,N'-methylenebisacrylamide, trimethylol propane triacrylate and triallyl amine. The crosslinking agent can generally constitute from about 0.001 mole percent to 5 mole percent of the resulting hydrogel-forming polymer material. More preferably, the crosslinking agent will constitute from about 0.01 mole percent to 3 mole percent of the hydrogel-forming polymeric gelling material.

The slightly crosslinked, hydrogel-forming polymeric gelling materials are generally employed in their partially neutralized form. For purposes of the present invention, such materials are considered partially neutralized when at least 25 mole per-cent, and preferably at least 50 mole percent of monomers used to form the polymer are acid group-containing monomers that have been neutralized with a salt-forming cation. Suitable salt-forming cations include alkali metal, ammonium, substituted ammonium and amines. This percentage of the total monomers utilized which are neutralized acid group-containing monomers is referred to herein as the "degree of neutralization."

While these absorbent gelling materials have typically been disclosed in the prior art in granular form, it is required by the present invention that the absorbent gelling material is in a non-granular form for example as macrostructures such as fibers, sheets or strips. These macrostructures can be prepared by forming the particulate absorbent gelling material into an aggregate, treating the aggregated material with a suitable crosslinking agent, compacting the treated aggregate to densify it and form a coherent mass, and then curing the compacted aggregate to cause the crosslinking agent to react with the particulate absorbent gelling material to form a composite, porous absorbent macrostructure. Such porous, absorbent macrostructures are disclosed, for example, in U.S. Patent 5,102,597 (Roe et al), issued April 7, 1992.

More generally any super-absorbent fiber known in the art may be used as a super-absorbent fiber in the absorbent core. Superabsorbent fibers can be made by forming a water soluble super-absorbent polymer into water soluble filaments, contacting the filaments with a primary air stream having a velocity effective to attenuate and to partially dry the filaments, and contacting the attenuated filaments with a secondary air stream having a velocity effective to fragment the filaments into fibers. Particularly suitable super-absorbent polymers are polymers comprising a blend of
- a copolymer of at least one alpha, beta-unsaturated carboxylic monomer and at least one monomer copolymerizable therewith, and
- a cross-linking agent having cross-linking functionality comprising hydroxyl or heterocyclic carbonated groups. Preferred are maleic anhydride/isobutylene copolymers cross-linked with propylene carbonate or a mixture of pentaerythriol and butanediol.

An example of a super-absorbent fiber embodiment of the present invention is Fibersorb, a commercially available superabsorbent fiber from Arco Chemical Company of Newton Square, Pennsylvania. These fibers are disclosed more fully in U.S. Patent No. 4,855,179, issued August 8, 1989, to Bourland et al.

### d. Optional Fibrous Layer

An optional component for inclusion in the absorbent cores according to the present invention is a fibrous layer adjacent to, and typically underlying the storage layer. This underlying fibrous layer would typically provide the same function as the secondary fluid distribution layer.

### e. Other Optional Components

The absorbent cores according to the present invention can include other optional components normally present in absorbent webs. For example, a reinforcing scrim can be positioned within the respective layers, or between the respective layers, of the absorbent cores. Such reinforcing scrims should be of such configuration as to not form interfacial barriers to fluid transfer, especially if positioned between the respective layers of the absorbent core. Given the structural integrity that usually occurs as a result of thermal bonding, reinforcing scrims are usually not required for the absorbent structures according to the present invention.

Another component which can be included in the absorbent core according to the invention and preferably is provided close to or as part of the primary or secondary fluid distribution layer are odor control agents. Typically active carbon coated with or in addition to other odor control agents, in particular suitable zeolite or clay materials, are optionally incorporated in the absorbent core. These components if present must also be incorporated in non-granular form.

### Physical characteristics of absorbent cores

Absorbent cores are usually non extensible and non-elastic, however, they can be rendered extensible and depending on the selected materials can also be made to have elastic characteristics. The term "extensible" as used hereinafter refers to a structure which under external forces such as those occurring during use of a thong liner extends in the direction of the forces or in the direction of a component of the forces in cases where only mono directional extensibility is provided.

The term "elastic" as used hereinafter refers to extensible structures which return at least partially to their initial state after the forces causing the extension seize to be excerted. Absorbent cores can be corrugated or pleated in one or several directions to provide a certain extensibility while selection of elastic fibers for the structure can provide elasticity.

The absorbent cores should preferably be thin. A thickness of less than 2.50 mm, preferably less than 2 mm is desirable such that the resulting thong liners have a thickness of 2.54 mm or less, preferably 2.032 mm or less.

Since the thinness of the thong liner is of key importance for comfort it is also important to eliminate as many discontinuities, at least within the plane of the thong liner. This leads to a preferable core design which extends to the periphery of the longitudinal sides and the rear end, even more preferable also to the front end of the thong liner. Preferably the thong liners are made from a continues band of a laminate comprising all materials, including the core. The laminate would then be cut to create individual thong liners.

It is then necessary that the core of the thong liner is composed in such a way that cutting the thong shape does not cause problems of loose materials becoming exposed from the core. As will be appreciated by those skilled in the art this is equally important for the benefit of the ultimate user of such thong liners but also from an industrial hygiene point of view during manufacturing.

Most preferably the thong liners are cut from a laminate band such that their shapes are oriented or meshed to produce as little throw away as possible. If this requires orienting the thong liners in direction differing from one thong liner to another than this would be the optimum cutting design.

The absorbent core provides the main component as far as pliability, stiffness, flexibility of the tong liner. It is therefore essential that the absorbent core does not create a barrier to the comfort of the thong liner by having too high a bending resistance or resistance to conforming to its in-use situation, including the changes occurring during use. This can be achieved by appropriate material selection and treatment of the core as will be readily apparent to the skilled person. For guidance the disclosure of the bending resistance measurement and requirements of 3807R can be used. It is however, particularly beneficial if the absorbent core is provided with a pre-scoring along its longitudinal centerline such that the garment facing surface of the absorbent core on the right and left side of the longitudinal centerline more readily fold towards each other (scoring would be on the garment facing side of the absorbent core). Even more preferred are in addition longitudinally symmetrical scoring lines offset from the longitudinal centerline, especially in the front half of the thong liner but on the wearer facing surface of the absorbent core. If the thong liner is made from a continues band of a laminate of all material then scoring can also be done on the complete product rather than just the core.

### Topsheet

The topsheet if present is compliant, soft feeling, and non-irritating to the wearer's skin. The topsheet also can have elastic characteristics allowing it to stretch in one or two directions. Further, the topsheet is fluid pervious permitting fluids (e.g., menses and/or urine) to readily penetrate through its thickness. A suitable topsheet can be manufactured from a wide range of materials such as woven and nonwoven materials; polymeric materials such as apertured formed thermoplastic films, apertured plastic films, and hydroformed thermoplastic films; porous foams; reticulated foams; reticulated thermoplastic films; and thermoplastic scrims. Suitable woven and nonwoven materials can be comprised of natural fibers (e.g., wood or cotton fibers), synthetic fibers (e.g., polymeric fibers such as polyester, polypropylene, or polyethylene fibers) or from a combination of natural and synthetic fibers.

Preferred topsheets for use in the present invention are selected from high loft nonwoven topsheets and aperture formed film topsheets. At least in the region where liquid is expected to be discharged onto the thong liner the apertured formed films are preferred because they are pervious to body exudates and yet non-absorbent and have a reduced tendency to allow fluids to pass back through and rewet the wearer's skin. Thus, the surface of the formed film that is in contact with the body remains dry, thereby reducing body soiling and creating a more comfortable feel for the wearer. Suitable formed films are described in U.S. Patent 3,929,135 (Thompson), issued December 30, 1975; U.S. Patent 4,324,246 (Mullane, et al.), issued April 13, 1982; U.S. Patent 4,342,314 (Radel. et al.), issued August 3, 1982; U.S. Patent 4,463,045 (Ahr et al.), issued July 31, 1984; and U.S. 5,006,394 (Baird), issued April 9, 1991. Particularly preferred microapetured formed film topsheets are disclosed in U.S. patent 4,609,518 (Curro et al), issue September 2, 1986 and U.S. patent 4,629,643 (Curro et al), issued December 16, 1986. One preferred topsheet for the present invention is the formed film described in one or more of the above patents and marketed on sanitary napkins by The Procter & Gamble Company of Cincinnati, Ohio as "DRI-WEAVE."

It is, however, particularly preferred that the topsheet be a so-called hybrid topsheet in which the wearer contacting surface is provided in its longitudinal center by an apertured formed film while a region not including the center is provided with a non-woven such as e.g. the high loft non-woven mentioned above or other non-woven which does provide particularly skin friendliness. Such topsheets have been disclosed in EPA-523 683, EP-A-523 719, EP-A-612 233, or EP-A-766 953.

Topsheets having not a homogeneous distribution of liquid passage ways but only a portion of the topsheet comprising liquid passage ways are also contemplated by the present invention. Typically such topsheets would have the liquid passage ways oriented such that they result in a centrally permeable and peripherally impermeable topsheet for liquids.

The body surface of the formed film topsheet can be hydrophilic so as to help liquid to transfer through the topsheet faster than if the body surface was not hydrophilic. In a preferred embodiment, surfactant is incorporated into the polymeric materials of the formed film topsheet such as is described in PCT Publication No. WO93/09741, "Absorbent Article Having A Nonwoven and Apertured Film Coversheet" filed on November 19, 1991 by Aziz, et al. Alternatively, the body surface of the topsheet can be made hydrophilic by treating it with a surfactant such as is described in the above referenced U.S. 4,950,254.

### Backsheet

The backsheet prevents the exudates absorbed and contained in the absorbent core from wetting articles that contact the thong liner such as string undergarments. Preferably the backsheet is impervious to liquids (e.g., menses and/or urine). It can be manufactured from a modified thin plastic film, although other flexible liquid impervious materials can also be used. As used herein, the term "flexible" refers to materials that are compliant and will readily conform to the general shape and contours of the human body. The backsheet preferably also can have elastic characteristics allowing it to stretch in one or two directions.

The thong liners according to the present invention have a breathable backsheet preferably comprising apertures.

The backsheet can comprise a woven or nonwoven material, polymeric films such as thermoplastic films of polyethylene or polypropylene, or composite materials such as a film-coated nonwoven material or fiber coated film. Preferably, the backsheet comprises a modified polyethylene film having a thickness of from about 0.012 mm (0.5 mil) to about 0.051 mm (2.0 mils).

The backsheet is preferably embossed and/or matte finished to provide a more clothlike appearance (unless the outer surface is provided by a woven or non-woven material as such). The backsheet can also comprise more than one breathable layer so as to replace a single breathable backsheet layer by at least 2 or 3 layers of a different or the same material. In particular two breathable layers forming together the breathable backsheet are preferred.

In an embodiment of the present invention the breathable backsheet comprises a hydrophobic, gas-permeable fibrous fabric layer composed of polymeric fibers, which is substantially liquid impervious. Such fibrous layers have inherent breathability apertures but in addition may be provided with apertures such as the film described below.

The fibrous fabric layer useful in the present invention preferably has a basis weight of 10 to 100 g/m² preferably 15 to 30 g/m². The fibres can be made of any hydrophobic polymeric material usual in the art of making fibrous fabric layers. Depending on the circumstances of the ultimate use and manufacturing of the breathable absorbent article fibres of polyethylene, polypropylene, polyester, polyacetat or combinations thereof (intra- and inter-fibres combinations) have been found useful.

The fibres are spunbonded, carded, melt blown or preferably combinations thereof. The fabric layer most preferably comprises a matrix of spunbonded fibres covered on one or both sides with meltblown fibres but can also be provided by any other typical technology used in the art. If two or more fibrous fabric layers of different fiber thickness are used it is most preferred to have the one with the higher fiber thickness on the outside of the thong liner

In an alternative embodiment of the present invention the breathable backsheet comprises a hydrophobic, gas-permeable apertured polymeric film. This film preferably has a first liquid transport direction and a second liquid transport direction opposite the first liquid transport direction and is oriented such that the first direction is from the backsheet towards the absorbent core. This directional apertured film allows a liquid transport in the first liquid transport direction which is larger than the liquid transport in the second liquid transport direction under an identical pressure drop across said apertured film. Most preferably the film comprises funnel shaped apertures wherein the direction from the larger funnel opening towards the smaller funnel opening is parallel to the direction from the garment facing side of the backsheet to the wearer facing side.

The apertured film also useful for embodiments of the present invention can be any of those well known in the art. This includes in particular, but is not limited to those films disclosed in U.S. 3,929,135, U.S. 4,151,240, U.S. 4,319,868, U.S. 4,324,426, U.S. 4,342,314 , U.S. 4,591,523 and U.S. 4,609,518, U.S. 4,629,643, U.S.5,158,819, U.S. 4,772,444.

The apertured film comprised in the inner layer of the breathable backsheet preferably has funnel shaped apertures similar to those described e. g. in US 3,929,135. The apertures maybe circular or non-circular but have a cross sectional dimension at one end of the funnel which is wider than the opening at the other end of the funnel. The direction from the larger funnel opening towards the smaller opening is of course generally parallel to the first liquid transport direction. The open area of the apertured film is typically more than 5 %, preferably in the range of 10 % to 35 % of the total film surface. The apertured films can be made of any material typical in the art but preferably is made of a polymer similar to those used for the fibrous fabric layer.

The minimum hydraulic diameter of the apertures in the film should be as small as possible while still providing sufficient gas permeability without hydraulic blockage of the apertures. A hydraulic diameter of as little as 0.1 mm, preferably 0.2 to 0.7 mm has been found possible in the context of the present invention. Hydraulic diameter for non circular apertures is the diameter that a circular aperture with the same cross section would have. Diameter is always determined in the plane of smallest cross section of an aperture.

As an alternative to the apertured film or non-woven backsheets (with or without a secondary backsheet layer) developments have been made to provide water vapour permeability to films, in particular to polyethylene films without creating apertures of a size sufficient to allow liquid transport. Such films provide liquid imperviousness (at least up to a minimum pressure gradient across the film) while allowing water vapour to permeate the film. Such films have been termed micro-porous films and are widely used in the disposable absorbent article industry for diaper backsheets, backsheets for sanitary napkins including panty liners.

Such micro-porous backsheets do not suffer from the particle loss phenomenon associated with apertured backsheets but nevertheless suffer in the context of some liners from the use of particles, especially large particle size materials. The reason is that this micro-porous materials are often very soft and pliable due to their manufacturing process in which conventionally a polyethylene is mixed with a calcium carbonate and the film thereafter is drawn such that the calcium carbonate, which is incompatible with the polyethylene, introduces locations where cracks are formed through the film such that these cracks form micro pores which allow water vapour to permeate through the film.

These particularly pliable and soft materials will, however, also transmit localized forced peaks such as can be created by particles. Therefore, the use of particles in the absorbent structures of thong liners using a micro-porous, water vapour permeable, breathable backsheet film could create discomfort due to the closeness of wearing such thong liners. Also, any particle pressing against the micro-porous backsheet may create a local aperture which is not desirable for backsheets.

Micro-porous films are well-known in the art and many disclosures of micro-porous films have been made. A list of processes for preparing micro-porous films and the use of micro-porous films in absorbent articles in general can be found for example in GB-A-1,452,977, US-4,347,844, US-4,091,164, US-4,153,751, US-4,698,372, US-4,815,714, US-4,923,650, US-5,008,296, US-5,011,698, US-5,409,761. Micro-porous films can be obtained for example from the Exxon Company, USA, or the Mitsui Company, Japan.

### Thong liner design

The thong liner as indicated above can be used beneficially in the context of sanitary napkins or panty liners. However especially thin thong shaped panty liners are particularly susceptible to the present invention. Sanitary napkins or panty liners having a thickness of 2.54 mm or less, preferably 2.032 mm or less, most preferably less than 1.2 mm, benefit especially well from the present invention.

For the following dimension definitions the thong liner has a front half, the portion 4 in Figure 1, and a rear half, the portion 5 in Figure 1, which are separated by transverse line 22. Transverse line 22 of Figure 1 is located at half the length o the thong liner (10) of Figure 1.

In order to define the width of the thong liner a theoretical linear longitudinal side edge (24), as shown in Figure 2, can be utilized. Where this line touches the periphery of the thong liner (10), namely in the front half at point 26 and in the rear half at point 25, the width perpendicular to the longitudinal center line (20) can be measured to define the relevant front and rear width of a thong liner.

The thong shape design is such that it provides the sanitary napkin or panty liner with a shape such that it can be worn in the above mentioned thong slips, G-string undergarments or string panties, hence the thong shape is fundamentally triangular or trapezoidal. The shapes of thong slips can still vary considerably but for practical purposes and in the context of the use of thong shaped panty liners or sanitary napkins will not have a rearward (or string) portion of less than 0.7 cm width.

In order for the design of the thong liner to properly fit into a thong slip while still providing a meaningful absorbency it is desirable that the front width of the thong liner is between 4 and 10 cm, preferably between 5 and 8 cm and most preferably between 5.5 and 6.5 cm. The rear width of the thong liner of course depends how far back it is intended to reach into the thong slip such that it could be as little as 0.7 cm. However, at this width it becomes very difficult for the wearer to manipulate the thong liner into the thong slip. It is therefore either preferred that the thong liner does not extend to a region in which the thong slip is so narrow or that the thong slip be wide enough for the thong liner to fit into it. The rearward width of the thong liner is preferably between 1.5 and 4 cm, more preferably between 1.8 and 2.5 cm. For thong liners which have rounded ends or which have a circular end it should be noted that the width requirement mentioned above has to be measured according to the above definition.

The points 25 and 26 where the linear line 24 touches the thong liner (10) in Figure 2 also define the end points of the linear or inwardly concave longitudinal side edge. The most preferred thong liners are selected such that they have a front width which is at least twice as wide as the rear width. Also preferably such thong liners have a length of 10 to 23 cm, preferably 12 to 16 cm, for thong shaped panty liners or 14 to 30 cm, preferably 19 to 25 cm, for thong shaped sanitary napkins. The front width of a thong liner is decisive for the placement in a thong slip since that width will be used by the wearer to decide positioning of the thong liner in general. Hence creating a particularly wide forward end with strong inward flaring longitudinal side edges can be helpful in creating an aid in positioning the thong liner.

In the following not limiting shape design examples are discussed which have been found particularly useful in the context of the present invention. It should be noted that all of them have inwardly concave longitudinal side edges since these are substantially preferred for good fit in thong slip undergarments, while linear edge designs are more preferred for ease of manufacturing when considering the high speed manufacturing of commodity articles such as the thong liners according to the present invention.

Figure 1 shows a thong shaped panty liner (10) having a longitudinal center line (20) and a transverse line (22) which separates a front half (4) of the thong shaped panty liner (10) and a rear half (5) of the panty liner (10). The panty liner further has a front end (12) and a rear end (14) together with 2 longitudinal side edges (16). As can be seen the side edges (16) are inwardly concave relative to a linear design. In this preferred embodiment the front edge (12) of the thong liner (10) also has an inwardly concave curvature which provides a so-called heart-shaped front end design.

In figure 2 the theoretical linear side edge of a linear design is shown by dashed-line (24). Also figure 2 shows the topsheet having a series of bonding locations (18) at which the topsheet is bonded at least partially to the respective underlying layer in line with the teaching of EP-A-617 602.

The thong liner (10) shown in figure 3 is shortened which is particularly useful for thong slips having an extremely thin rearward string portion.

### Optional components of the absorbent thong liners

Optionally, the thong liner of the present invention can comprise all those components typical for the intended product use. For example thong liners can comprise components such as wings in order to improve their positioning and soiling protection performance especially towards the rear end of the article.

Such design are shown for example in US design 394,503, UK designs 2,076,491 and 2,087,071 as well as internationally filed industrial model DM 045544, filed under the Hague Agreement, registered on October 21, 1998. Particularly for thong shaped sanitary napkins it can be desirable to have wings extending substantially over the complete rear half of the napkin and even partially into the front half. Such wings would then be folded onto the external side of a thong slip. In the central portion of the thong shaped sanitary napkin the wings would fold onto the external crotch portion of the slip while in the rear ward portion of the slip, in the string region, the wings would embrace the string and overlap each other. It is further desirable that such wings are provided with an adhesive to attach them to each other and to the underlying slip cloth, as is usual with conventional wings.

Irrespective of the wings and whether they are specially designed for thong shaped sanitary napkins or for thong shaped panty liners they can be provided as separate pieces and be attached to the thong liner or they can be integral with the materials of the thong liner, e.g. by being integral extension of the topsheet, the backsheet or a combination thereof. If the wings are attached then they can be attached in a basic outward pointing position or already be predisposed towards their in-use position, i.e. towards the longitudinal centerline. If the wings are attached then the longitudinal side edges of the thong liner remain clearly defined and the requirement of a linear or inwardly concave shape can easily be assessed. If the wings are integral then the requirement of a linear or inwardly concave shape for the longitudinal side edges is provided by the main body portion of the thong liner. The main body portion, or at least the longitudinal side edge thereof is given by the core shape and can be assessed as the line closest to the core along which the wings will bend when exposed to a pivoting pressure (holding the core portion of the thong liner and pressing (lightly) on the external wing edge). In either case (attached wings or integral wings) the wings are not included in assessing the longitudinal side edge shape of the thong liners.

In general, all typically used components in absorbent products can also be comprised in the thong liners according to the present invention as long as the article comprises a breathable backsheet and a non-granular super-absorbent material.

Most preferred thong liners will comprise a panty fastening adhesive for attachment to the thong slip. The design of the fastening adhesive must be selected such that it does not interfere with the desired breathability while at the same time such adhesive should ensure proper fit. Distributed adhesives such as spray filaments (random location) or printed dots (designed location) have been found useful. Alternatively, the adhesive can be water vapour pervious as such. Beyond this the above mentioned limitations on adhesive coverage, especially in the rear half of the thong liners are referred to.

## Claims

1. Disposable breathable absorbent sanitary napkin or panty liner (10) having a wearer facing surface and a garment facing surface, and comprising
an absorbent core comprising a fibrous matrix, and
having a front end (12) and a rear end (14) wherein the rear end (14) is directed towards the rear of a wearer during use, said sanitary napkin or panty liner (10) having a backsheet on said garment facing surface,
said backsheet and said absorbent core being water vapour permeable, preferably air permeable,
said sanitary napkin or panty liner (10) having a thong shape, said thong shape being such that said front width of said sanitary napkin or panty liner (10) is at least twice as wide as said rear width and the longitudinal side edges (16) connecting the front and rear end of the sanitary napkin or panty liner (10) have a linear or inwardly concave contour,
and said sanitary napkin or panty liner (10) being **characterized in that** said core further comprises 5% to 95%, preferably 5% to 50%, most preferably 8% to 35%, fibrous super absorbent material by weight of said absorbent core.

2. Sanitary napkin or panty liner (10) according to claim 1 comprising on said garment facing surface of said backsheet a panty fastening adhesive covering at least 75 % of the surface of said backsheet.

3. Sanitary napkin or panty liner (10) according to any of the preceding claims **characterized in that** said absorbent core extends to the periphery of said longitudinal side edges (16) and said rear end (14), and preferably said core extends completely throughout the sanitary napkin or panty liner (10).

4. Sanitary napkin or panty liner (10) according to any of the proceeding claims **characterized in that** they also comprise a liquid and water vapour pervious topsheet, preferably provided at least partially by an apertured thermo-plastic film.

5. Sanitary napkin or panty liner (10) according to any of the proceeding claims **characterized in that** the thickness of the napkin or liner (10) is less than 2.54 mm, preferably less than 2.032 mm.
